# EUROPÄISCHE PATENTSCHRIFT

(11) **EP 0 912 197 B1**
(45) Veröffentlichungstag und Bekanntmachung des Hinweises auf die Patenterteilung: **05.12.2001**
(21) Anmeldenummer: 97931768.2
(22) Anmeldetag: 07.07.1997
(51) Int. Cl.: A61K 47/48

(54) **HÄMOGLOBIN-HYDROXYETHYLSTÄRKE-KONJUGATE ALS SAUERSTOFF-TRANSPORT-MITTEL**
HAEMOGLOBIN-HYDROXYETHYL STARCH CONJUGATES AS OXYGEN CARRIERS
CONJUGUES HEMOGLOBINE-AMIDON HYDROXYETHYLE COMME AGENTS DE TRANSPORT D'OXYGENE

(30) Priorität: 08.07.1996 DE 19628705
(43) Veröffentlichungstag der Anmeldung: 06.05.1999
(73) Patentinhaber: Fresenius AG, 61440 Oberursel (DE)
(72) Erfinder: SOMMERMEYER, Klaus, D-61191 Rosbach (DE); EICHNER, Wolfram, D-35510 Butzbach (DE)
(74) Vertreter: UEXKÜLL & STOLBERG
(86) Internationale Anmeldenummer: EP9703527
(87) Internationale Veröffentlichungsnummer: WO9801158

(56) Entgegenhaltungen:
- EP-A- 0 338 916
- DE-A- 3 029 307
- US-A- 4 064 118

## Beschreibung

Die vorliegende Erfindung betrifft neue Sauerstoff-Transport-Mittel, die Hämoglobin-Hydroxyethylstärke-Konjugate enthalten, sowie Verfahren zu deren Herstellung. Die Erfindung betrifft ferner die Verwendung der Sauerstoff-Transport-Mittel als Blutersatzstoff, Plasma-Expander, Perfusionsmitttel, Hämodilutionsmittel und/oder kardioplegische Lösung.

Die Entwicklung von stromafreien Hämoglobin-Lösungen, sogenannten "Hämoglobin-Based-Oxygen Carriers" (HBOC's), die als Blutersatzstoff verwendbar sind, ist seit langem ein vordringliches Ziel der pharmazeutischen Forschung und Entwicklung.

Blutverlust, beispielsweise als Folge eines Unfalls oder einer Operation, wird in den meisten Fällen mit einer allogenen Blutspende behandelt. Die damit assozierten Probleme des unkontrollierten Transfers von pathogenen Organismen, insbesondere von Viren wie HIV oder Hepatitis-Erregern, sowie die Notwendigkeit der Blutgruppentypisierung vor der Transfusion sind dem Fachmann seit langem bekannt und in der Literatur umfassend beschrieben.

Ein als vollwertiger Blutersatz verwendbares HBOC-Produkt würde nicht nur diese Probleme lösen, sondern könnte darüber hinaus als Plasma-Expander, Perfusionsmittel, Hämodilutionsmittel und/- oder kardioplegische Lösung verwendet werden.

Obgleich der Bedarf an einem derartigen Produkt bereits frühzeitig erkannt wurde (vgl. Rabiner, J. Exp. Med. 126, (1967) 1127), hat bisher keines der bekannten HBOC-Produkte den Status eines zugelassenen Arzneimittels erreicht.

Das natürliche Sauerstoff-Transport-Mittel ist der Blutfarbstoff Hämoglobin, ein Chromoprotein mit einem Molekulargewicht (MG) von 64 Kilodalton (kDa). Das Protein besteht aus zwei α- und β-Peptidketten, die als prosthetische Gruppe jeweils ein Häm gebunden haben. Dabei handelt es sich um einen Porphyrin-Ring mit zentralem Eisen-Atom. Isolierte Hämoglobin-Moleküle sind sehr instabil und zerfallen rasch in die stabileren α,β-Dimere (MG 32 kDa). Die biologische Halbwertszeit von isoliertem Hämoglobin im Blutkreislauf liegt bei etwa 1 Stunde, da die Dimere schnell über die Nieren ausgeschieden werden. Dabei erzeugen die Dimere nephrotoxische Nebenwirkungen (vgl. Bunn & Jandl, J. Exp. Med. 129, (1967) 925-934).

Die zunächst entwickelten HBOC-Produkte besaßen zudem ein nephrotoxisches Potential, das auf Verunreinigungen der Produkte mit zellulären Bestandteilen zurückgeführt wurde (vgl. Relihan, Ann. Surg. 176, (1972) 700).

Ferner fehlt einer isolierten Hämoglobin-Zusammensetzung das 2,3-Diphosphoglycerat (2,3-DPG), welches den natürlichen, allosterischen Aktivator der Sauerstoffbindung darstellt. Daraus resultiert eine gesteigerte Sauerstoff-Bindungsaffinität des isolierten Hämoglobins und, damit einhergehend, ein verringertes Sauerstoff-Freisetzungsvermögen derartiger Zusammensetzungen.

Entwicklungsarbeiten an derivatisierten Hämoglobin-Molekülen waren daher in erster Linie darauf gerichtet, deren Sauerstoffübertragungseigenschaften zu verbessern, sowie die nephrotoxischen Symptome zu umgehen. Dabei wurde Hämoglobin intramolekular vernetzt, zur Bildung von polymeren HBOC-Formen intermolekular verknüpft und/oder an Polymere gekoppelt, um konjugierte HBOC-Formen zu schaffen. Dabei wurden auch Mischformen von intra- und intermolekular vernetzten Hämoglobin-Derivaten hergestellt und auf die geplante Verwendung hin untersucht.

Die Vernetzung von Hämoglobin mittels bi- oder polyfunktionaler Vernetzungsmittel kann selektiv oder nicht-selektiv erfolgen. Bei einer Form der selektiven Vernetzung werden zwei Proteinketten des Hämoglobins intramolekular miteinander verbunden, wodurch die natürliche tetramere Form des isolierten Hämoglobin-Moleküls stabilisiert wird. Durch Auswahl eines geeigneten Vernetzungsmittels kann die Sauerstoff-Affinität des Hämoglobins ferner so eingestellt werden, daß vernetztes Hämoglobin unter physiologischen Bedingungen Sauerstoff reversibel binden kann. Beispiele für derartige Vernetzungsmittel sind Pyridoxalphosphat und Diaspirin sowie deren Derivate. Verfahren zur Vernetzung von Hämoglobin werden beispielsweise in Benesch (Meth. Enzymol., Vol. 231 (1994), 267-274), Keipert et al. (Transfusion, Vol. 29 (1989), 767-773), Snyder et al. (Proc. Natl. Acad. Sci. USA, 84 (1987), 7280-7284) und in Rogers et al. (Biochim. et Biophys. Acta, 1248 (1995), 135-142) beschrieben.

Bei einer nicht-selektiven Vernetzung entstehen intermolekular vernetzte, polymere HBOC-Produkte. Entsprechende Vernetzungsmittel und Verfahren zu deren Verwendung werden beispielsweise in DE-26 07 706, EP-0 646 130 und Hai et al. (Art. Cells, Blood Subs. and Immob. Biotech, 22(3) (1994), 923-931) beschrieben. Einen Überblick über verschiedene Hämoglobin-Derivate und die mit dem klinischen Einsatz verbundenen Probleme geben die Veröffentlichungen von Gould et al., Transfus. Sci. 16, (1995) 5-17, und Chang et al., Biomat., Art. Cells & Immob. Biotech., 20, (1992) 159-179.

Bekannte Hämoglobin-Konjugate werden umfassend in Xue und Wong (Meth. in Enzymol., 231 (1994), S. 308-322) und beispielsweise in DE 26 16 086 oder DE 26 46 854 beschrieben. Letztere offenbart Verfahren mittels derer Hämoglobin an Hydroxyethylstärke gebunden wird, indem Hydroxyethylstärke zunächst mit Natriumperiodat umgesetzt wird. Dabei entstehen Dialdehyde, an die Hämoglobin gebunden wird. Demgegenüber beschreibt die DE 26 16 086 die Kopplung von Hämoglobin an Hydroxyethylstärke nach einem Verfahren, bei dem zunächst ein Vernetzungsmittel (z.B. Bromcyan) an die Hydroxyethylstärke gebunden wird und anschließend: Hämoglobin an das Zwischenprodukt gebunden wird.

Die Sauerstoff-Bindungsaffinität der Hämoglobin-Derivate hängt neben der Auswahl von geeigneten Vernetzungs- und/oder Polymerisationsmitteln auch vom Liganden der Häm-Gruppe während der Vernetzung und/oder Polymerisation ab. Oxy-Hämoglobin oxidiert schnell zu Met-Hämoglobin (Fe-III), welches eine zu hohe Sauerstoff-Bindungsaffinität besitzt, um als Sauerstoff-Transport-Mittel geeignet zu sein. Daher wurden die genannten Verfahren zur Herstellung von HBOC-Derivaten auch mit teilweise oder vollständig desoxygeniertem Hämoglobin durchgeführt (vgl. Benesch, R. E. a.a.O.).

Die bisherigen Verfahren zur Herstellung von vernetzten und/oder konjugierten HBOC-Produkten ermöglichten jedoch keine selektive Bindung von Hämoglobin an das jeweilige Polymer. Es entstand in allen Verfahren ein Gemisch von Co-Polymeren, dessen Bestandteile unterschiedliche biologische Aktivitäten aufwiesen. Das Reaktionsprodukt, bzw. die Zusammensetzung der Mischung konnte bislang nur grob charakterisiert werden. Sowohl die höhermolekularen Produkte (MG > 500 kDa) als auch residuale, tetramere Formen führten zu toxischen Nebenwirkungen. Die Entfernung der jeweiligen nieder- und/oder hochmolekularen Anteile aus den HBOC-Produkten, zum Beispiel durch zusätzliche Filtrationsschritte, bedingt beträchtliche Ausbeuteverluste, wodurch die Wirtschaftlichkeit der Herstellungsverfahren erheblich verschlechtert wird.

Die bisher getesteten HBOC-Produkte wiesen zusätzlich vaskuläre Nebenwirkungen auf, die nach neuesten klinischen Studien auf niedermolekulare, d.h. im wesentlichen tetramere HBOC-Formen zurückzuführen sind (vgl. Gould et al., a.a.O., und Alayash & Cashon, Molecular Medicine Today, 1, (1995) 122-127). Diese niedermolekularen HBOC-Formen sind in der Lage, aus dem Blutkreislauf in die endothelialen Zellagen der Blutgefäße überzutreten. Die hohe Bindungsaffinität des Hämoglobin für das Stickoxid (NO, auch bekannt als Endothelial-Derived Relaxing Factor, EDRF) führt dazu, daß sich die frei verfügbare NO-Menge in diesem Gewebe nach Applikation von HBOC-Derivaten drastisch reduziert. Als Folge der lokalen Verringerung der NO-Konzentration entsteht eine systemische Vasokonstriktion, die zu Hypertonie führt.

Jia et al. (Nature, 380, (1996) 221-226) schreiben dem Hämoglobin sogar eine zentrale Rolle in der Regulation des NO-Kreislaufs zu. Demnach wird Hämoglobin in der Lunge kooperativ oxygeniert und S-nitrosiliert. Die NO-Gruppe wird während des arterio-venösen Übergangs auf andere Proteine übertragen, welche dadurch eine NO-ähnliche, gefäßerweiternde Aktivität erlangen. Vernetzte HBOC-Produkte besitzen jedoch in der Regel keine kooperativen Eigenschaften mehr.

Eine weitere toxische Aktivität der bisher getesteten HBOC-Produkte wurde u.a. von Alayash und Cashom beschrieben (vgl. Molec. Med. Today, (1995) a.a.O.). Demnach sind Hämoglobin-Moleküle außerhalb der Erythrozyten an Redoxreaktionen beteiligt, in deren Verlauf hochreaktive Hämoglobin- und Sauerstoff-Spezies entstehen, die u.a. für Lipid-Peroxidation verantwortlich gemacht werden.

Um die toxischen Nebenwirkungen der bislang getesteten HBOC-Produkte zu unterbinden, wurden Applikationsformen entwickelt, bei denen Hämoglobin in Liposomen verpackt wird, wodurch künstliche, Erythrozyten-ähnliche Transportorganellen für Hämoglobin entstehen (vgl. Rudolph et al., Crit. Care Med. 22, (1994) 142-150). Der hohe Eintrag von Phospholipiden in den Blutkreislauf ist jedoch mit einem weiteren Risiko für die Patienten verbunden.

zusammenfassend kann festgehalten werden, daß die bisher getesteten HBOC-Produkte keine Arzneimittelzulassung erhalten haben, da deren klinische Verwendung als Sauerstoff-Transport-Mittel bisher durch eine unzureichende Verträglichkeit verhindert wurde.

Aufgabe der vorliegenden Erfindung war es daher, ein Sauerstoff-Transport-Mittel zur Verfügung zu stellen, welches als Blutersatzstoff klinisch verwendbar ist. Eine weitere Aufgabe dieser Erfindung liegt in der Bereitstellung eines geeigneten Herstellungsverfahrens für das erfindungsgemäße Sauerstoff-Transport-Mittel.

Diese Aufgabe wird durch ein Sauerstoff-Transport-Mittel gelöst, welches ein Hämoglobin-Hydroxyethylstärke-Konjugat enthält, in dem Hämoglobin und die Hydroxyethylstärke selektiv über Amidbindungen zwischen freien Aminogruppen des Hämoglobins und der in oxidierter Form vorliegenden reduzierenden Endgruppe der Hydroxyethylstärke miteinander verknüpft sind.

Es hat sich überraschenderweise gezeigt, daß erfindungsgemäße Hämoglobin-Hydroxyethylstärke-Konjugate hervorragend als Sauerstoff-Transport-Mittel geeignet sind, da diese besonders gut verträglich sind. Das Mittel weist eine Sauerstoffbindungsaffinität auf, die eine reversible Sauerstoff-Bindung unter physiologischen Bedingungen ermöglicht (P₅₀ von 20 bis 80 mm Hg, vorzugsweise von 20 bis 50 mm Hg; wobei die Bestimmung des P₅₀ bei maximaler Anreicherung mit reinem Sauerstoff - mindestens 150 mm Hg - erfolgt). Das Hämoglobin-Hydroxyethylstärke-Konjugat ist zu groß, um in die endothelialen Zellagen der Blutgefäße einzudringen, und verursacht daher keine hypertonischen Nebenwirkungen. Das Sauerstoff-Transport-Mittel enthält weder antigene noch pyrogene Bestandteile und verursacht auch keine nephrotoxischen Nebenwirkungen.

Erfindungsgemäß wurde überraschenderweise festgestellt, daß die vorteilhaften rheologischen Eigenschaften, die Hydroxyethylstärke zu einem bevorzugten Mittel für die Hämodilution und zum Volumenersatz gemacht haben (vgl. Weidler et al., Arzneim.-Forschung/ Drug Res., 41, (1991) 494-498), in dem Konjugat erhalten bleiben. Die gute Verträglichkeit des Sauerstoff-Transport-Mittels begründet sich somit auch durch eine überraschende Kombination der vorteilhaften Sauerstoff-Transport-Eigenschaften des Hämoglobins und der Hämodilutions-Eigenschaften der Hydroxyethylstärke.

Das Sauerstoff-Transport-Mittel weist eine lange vaskuläre Persistenz auf und die Moleküloberfläche des Hämoglobins wird durch Substituenten abgeschirmt. Überraschenderweise wurde festgestellt, daß das Hämoglobin im erfindungsgemäßen Hämoglobin-Hydroxyethylstärke-Konjugat durch diesen Abschirmeffekt daran gehindert wird, an toxischen Redoxreaktionen teilzunehmen.

Ein weiterer Vorteil des erfindungsgemäßen Sauerstoff-Transport-Mittels besteht darin, daß Hydroxyethylstärke und Hämoglobin als Konjugat gleichzeitig in hohen Konzentrationen verabreicht werden können, ohne daß dadurch der kolloid-osmotische Druck erhöht wird.

Das Sauerstoff-Transport-Mittel enthält das Hämoglobin-Hydroxyethylstärke-Konjugat in einer Konzentration zwischen 2 und 40 g/dL, bevorzugt in einer Konzentration zwischen 5 und 20 g/dL und besonders bevorzugt in einer Konzentration von 8 bis 20 g/dL. Das Sauerstoff- Transport-Mittel kann ferner bekannte physiologisch verträgliche Träger, Verdünnungsmittel oder Exzipienten enthalten.

Im Rahmen der vorliegenden Erfindung wird zur Herstellung des Hämoglobin-Hydroxyethylstärke-Konjugates bevorzugt stroma-freies, gereinigtes und pasteurisiertes Hämoglobin verwendet, das nach im Stand der Technik umfassend beschriebenen Verfahren gewonnen werden kann. Das Hämoglobin kann vernetzt und/oder polymerisiert sein. Das Hämoglobin kann menschlichen, tierischen, pflanzlichen oder rekombinanten Ursprungs sein. Im Rahmen der vorliegenden Erfindung wurde überraschenderweise festgestellt, daß die Abschirmeffekte der Hydroxyethylstärke immunologische Komplikationen verhindern, die bei der Verwendung von tierischem Hämoglobin zu erwarten wären. Eine bevorzugte Ausführungsform der Erfindung betrifft daher ein Sauerstoff-Transport-Mittel, welches ein Hämoglobin-Hydroxyethylstärke-Konjugat enthält, in dem das Hämoglobin tierischen Ursprungs ist. Das Hämoglobin kann beispielsweise bovinen, porcinen oder equinen Ursprungs sein. Gemäß einer besonders bevorzugten Ausführungsform der Erfindung wird für die Herstellung des Hämoglobin-Hydroxyethylstärke-Konjugates bovines Hämoglobin verwendet, das in isolierter Form auch ohne Vernetzung die bevorzugte Sauerstoff-Bindungsaffinität aufweist.

Sofern humanes Hämoglobin verwendet wird, sollte dieses mittels Vernetztung und/oder Polymerisation in der tetrameren Form stabilisiert werden. Durch Vernetzung und/oder Polymerisation wird humanes Hämoglobin gleichzeitig zur reversiblen Sauerstoff-Bindung unter physiologischen Bedingungen befähigt. Dem Fachmann sind eine Vielzahl von Verfahren zur Vernetzung oder Polymerisation bekannt. Erfindungsgemäß kann ein beliebiges Verfahren verwendet werden, sofern das Hämoglobin dabei stabilisiert wird und die gewünschte Sauerstoff-Affinität (P₅₀ von 20 bis 80 mm Hg, bevorzugt P₅₀ von 20 bis 50 mm Hg) erhält. Bevorzugte Vernetzungsverfahren umfassen die intramolekulare Vernetzung mit bispyridoxal-Tetraphosphat (vgl. Keipert et al., Transfusion, Vol. 29 (1989), 767-773) oder Diaspirin (vgl. Snyder et al., Proc. Natl. Acad. Sci. USA, 84 (1987), 7280-7284) oder eine Vernetzung und Polymerisation mit oxidierter Raffinose (vgl. EP-0 646 130).

Gemäß einer besonders bevorzugten Ausführungsform der Erfindung liegt das Hämoglobin vor der Kopplung an die Hydroxyethylstärke in desoxygenierter oder teilweise desoxygenierter Form vor. Bei teilweise desoxygenierten Formen sind Zusammensetzungen bevorzugt, die zu 20 bis 80% aus Desoxy-Hämoglobin und zu 80 bis 20% aus Hämoglobin in anderen Derivat-Zuständen bestehen, wobei Zusammensetzungen aus 50 bis 80% Desoxy-Hämoglobin und 50 bis 20% Hämoglobin in anderen Derivat-Zuständen besonders bevorzugt sind. Mit anderen Derivat-Zuständen von Hämoglobin werden hier insbesondere CO-, Oxy- und/oder Met-Hämoglobin-Derivate bezeichnet.

Für die Herstellung des Konjugates wird vorzugsweise Hydroxyethylstärke verwendet, die ein mittleres Molekulargewicht von 1 bis 40 kDa aufweist, wobei Hydroxyethylstärke mit einem mittleren Molekulargewicht von 2 bis 20 kDa bevorzugt und Hydroxyethylstärke mit einem mittleren Molekulargewicht von 5 bis 20 kDa besonders bevorzugt ist. Bevorzugte Hydroxyethylstärke ist ferner durch einen molaren Substitutionsgrad von 0,1 bis 0,8 und ein Verhältnis von C₂ : C₆-Substitution im Bereich von 2 bis 20 charakterisiert.

Erfindungsgemäß bevorzugte Hydroxyethylstärke kann durch Säurehydrolyse, beispielsweise mit HCl, aus einer im Handel (Sigma) erhältlichen Hydroxyethylstärke mit vergleichsweise höherem Molekulargewicht gewonnen werden. Die Hydroxyethylstärke wird anschließend einer Fällungsreaktion unterworfen, wobei beispielsweise Aceton verwendet werden kann.

Das Molekulargewicht des erfindungsgemäßen Hämoglobin-Hydroxyethylstärke-Konjugates hängt von dem Molekulargewicht oder der Molekulargewichtsverteilung des eingesetzten Hämoglobins, der Molekulargewichtsverteilung der eingesetzten Hydroxyethylstärke und der Auswahl der Reaktionsbedingungen ab. Erfindungsgemäß werden Hämoglobin-Hydroxyethylstärke-Konjugate bevorzugt, deren Molekulargewicht zwischen 100 und 700 kDa liegt, wobei ein Molekulargewicht zwischen 200 und 300 kDa besonders bevorzugt ist.

Im Rahmen der vorliegenden Erfindung wurde festgestellt, daß die bekanntermaßen stabilisierende Wirkung von Sacchariden auf Hämoglobin (vgl. Rudolph, Cryobiology, 25, (1988) 1-8) auch von der Hydroxyethylstärke des Konjugates ausgeht, wenn kurzkettige Hydroxyethylstärke verwendet wird. Erfindungsgemäße Sauerstoff-Transport-Mittel weisen gegenüber unmodifizierten HBOC-Produkten somit eine verbesserte Lagerstabilität bei 4 °C und bei Raumtemperatur auf. Damit ist das Sauerstoff-Transport-Mittel überraschenderweise selbst zum Träger der vorteilhaft stabilisierenden Eigenschaften der Saccharide geworden.

Gegenstand der vorliegenden Erfindung sind auch die Verfahren zur Herstellung der Sauerstoff-Transport-Mittel, die ein Hämoglobin-Hydroxyethylstärke-Konjugat enthalten. Diese Verfahren ermöglichen erstmals eine selektive Bindung von Hämoglobin an Hydroxyethylstärke, wodurch ein Sauerstoff-Transport-Mittel entsteht. Das Konjugat wird in einem mehrstufigen Verfahren hergestellt, bei dem man die reduzierenden Endgruppen von Hydroxyethylstärke zunächst oxidiert und anschließend Hämoglobin über freie Aminogruppen mittels Amidbindungen an die oxidierten Endgruppen der Hydroxyethylstärke koppelt.

Als Ausgangsmaterial für das Verfahren wird vorzugsweise Hydroxyethylstärke mit einem mittleren Molekulargewicht von 1 bis 40 kDa verwendet, wobei Hydroxyethylstärke mit einem mittleren Molekulargewicht von 2 bis 20 kDa bevorzugt und Hydroxyethylstärke mit einem mittleren Molekulargewicht von 5 bis 20 kDa besonders bevorzugt ist, Bevorzugte Hydroxyethylstärke ist ferner durch einen molaren Substitutionsgrad von 0,1 bis 0,8 und ein Verhältnis von C₂ : C₆-Substitution im Bereich von 2 bis 20 charakterisiert.

Im Rahmen der Erfindung wird für die Herstellung des Sauerstoff-Transport-Mittels bevorzugt stroma-freies, gereinigtes, pasteurisiertes, vernetztes und/oder polymerisiertes Hämoglobin verwendet. Das Hämoglobin kann dabei menschlichen, tierischen, pflanzlichen oder rekombinanten Ursprungs sein. Im Rahmen der vorliegenden Erfindung wird bovines Hämoglobin bevorzugt, da es in isolierter Form eine Sauerstoff-Bindungsaffinität aufweist, die eine reversible Sauerstoff-Bindung unter physiologischen Bedingungen ermöglicht.

Nach einem bevorzugten Verfahren der Erfindung werden die reduzierenden Endgruppen der Hydroxyethylstärke oxidiert, indem man die Hydroxyethylstärke zunächst mit einer Iod enthaltenden Lösung vermischt und danach Kaliumhydroxyd-Lösung dazugibt.

Nach einem weiteren bevorzugten Verfahren der Erfindung wird das Hämoglobin in einem zweiten Schritt an die oxidierten Endgruppen von Hydroxyethylstärke gebunden. Die Reaktion kann beispielsweise durch Vermischen der Einzelkomponenten bei 40°C durchgeführt werden. Dabei kommt es zu einer nukleophilen Substitutionsreaktion zwischen einer freien Aminogruppe des Hämoglobins und dem Lacton der Hydroxyethylstärke, wobei eine Amidbindung entsteht, durch die Hämoglobin an die oxidierte reduzierende Endgruppe der Hydroxyethylstärke gebunden wird.

Erfindungsgemäß hat es sich demgemäß überraschenderweise gezeigt, daß nach dem Verfahren von Hashimoto et al. (Kunststoffe, Kautschuk, Fasern, 9, (1992) 1271-1279) zur Herstellung von Block-Copolymeren aus Polysacchariden und Polyamiden, gereinigtes Hämoglobin, das intra- und/oder intermolekular vernetzt sein kann, so an oxidierte Hydroxyethylstärke gebunden werden kann, daß ein besonders verträgliches Sauerstoff-Transport-Mittel entsteht. Unter Verwendung der erfindungsgemäßen Lehre läßt sich die Synthese eines Hämoglobin-Konjugates erstmals soweit kontrollieren, daß tetramere Hämoglobin-Formen an Hydroxyethylstärke gebunden werden, ohne daß ein nennenswerter Anteil an unerwünschten hochmolekularen Hämoglobin-Formen entsteht.

Gemäß einer bevorzugten Ausführungsform der Erfindung werden die Reaktionsbedingungen so ausgewählt, daß ein Hämoglobin-Hydroxyethylstärke-Konjugat entsteht, welches ein Molekulargewicht zwischen 80 und 800 kDa aufweist, wobei ein Molekulargewicht zwischen 100 und 500 und insbesondere zwischen 150 und 400 kDa bevorzugt ist.

Nach dem erfindungsgemäßen Herstellungsverfahren erfolgt eine annähernd quantitative Umsetzung des Hämoglobins mit der Hydroxyethylstärke. Es verbleiben somit auch kaum niedermolekulare Hämoglobin-Formen im Reaktionsansatz, wobei ein Gehalt von weniger als 5% an nicht konjugierten Hämoglobin-Formen bevorzugt ist. Demgemäß werden in einer weiteren bevorzugten Ausführungsform der Erfindung nach der Kopplung von Hämoglobin und Hydroxyethylstärke keine aufwendigen Reinigungverfahren benötigt, um das gewünschte Reaktionsprodukt zu isolieren.

Gemäß einer weiteren bevorzugten Ausführungsform der Erfindung liegt das Hämoglobin vor der Kopplung an die Hydroxyethylstärke entweder in vollständig desoxygenierter oder teilweise desoxygenierter Form vor. Bei einer teilweise desoxygenierten Form sind Zusammensetzungen bevorzugt, die zu 20 bis 80% aus Desoxy-Hämoglobin und zu 80 bis 20% aus Hämoglobin in anderen Derivat-Zuständen bestehen, wobei Zusammensetzungen aus 20 bis 80% Desoxy-Hämoglobin und 80 bis 20% Hämoglobin in anderen Derivat-Zuständen besonders bevorzugt sind.

Die Desoxygenierung des Hämoglobins kann mittels beliebiger chemischer oder physikalischer Verfahren erfolgen. Dabei wird Hämoglobin entweder mit chemischen Reduktionsmitteln wie Na-Ascorbat, Glutathion, N-Acetyl-Cystein oder N-Acetyl-Methionin versetzt oder mittels einer Gas-durchlässigen Membran gegen inertes Gas, wie N₂, He oder Ar, zirkuliert.

In einem besonders bevorzugten Verfahren wird Cystein oder Acetyl-Cystein als Reduktionsmittel verwendet. Die Reduktion wird durchgeführt, bis der Oxy-Hämoglobin-Gehalt weniger als 5% beträgt, wobei ein Gehalt von weniger als 1% bevorzugt ist. Der Gehalt an Met-Hämoglobin sollte weniger als 5% betragen, wobei ein Gehalt von weniger als 3 oder 1% und insbesondere ein Gehalt von weniger als 0,5% bevorzugt ist.

Gemäß einer weiteren besonders bevorzugten Ausführungsform der Erfindung wird für die Herstellung des Hämoglobin-Hydroxyethylstärke-Konjugates eine Hämoglobin-Lösung verwendet, in der das Hämoglobin zu 20 bis 80% aus Desoxy-Hämoglobin und zu 80 bis 20% aus Hämoglobin in anderen Derivat-Zuständen besteht. Zur Herstellung einer derartigen Hämoglobin-Lösung kann Oxy-Hämoglobin teilweise desoxygeniert werden oder Desoxy-Hämoglobin teilweise oxygeniert werden. Entsprechend der Derivat-Form der Ausgangs-Hämoglobin-Lösung sowie der gewünschten und erfindungsgemäß bevorzugten Hämoglobin-Derivat-Zusammensetzung kann eine Hämoglobin-Lösung ferner entweder mit Kohlenmonoxid-Gas in die stabile CO-Form überführt und/oder mit Sauerstoff oder O₂-haltigen Gasen oxygeniert und/oder mit Stickstoff oder anderen Inertgasen desoxygeniert werden. Der Gas-Austausch kann dabei nach beliebigen Stand der Technik beschriebenen Verfahren durchgeführt werden. Bevorzugte Verfahren umfassen die Begasung einer Desoxy-Hämoglobin-Lösung mit Sauerstoff oder mit einem Gas, das Sauerstoff enthält, oder eine chemische Teil-Reduktion des Oxy-Hämoglobins mit einem Reduktionsmittel, wie beispielsweise Na-Dithionat, Na-Ascorbat oder Na-Bisulfit.

Nach Abschluß der Reaktion kann das Reduktionsmittel beispielsweise durch Ultrafiltration abgetrennt werden. In einer bevorzugten Ausführungsform der Erfindung wird die Ultrafiltration mittels einer Membran durchgeführt, die das gewünschte Produkt im Retenat zurückhält.

Nach einem besonders bevorzugtem Verfahren der Erfindung wird das Hämoglobin anschließend unter N₂-Begasung lyophilisiert.

In einer weiteren besonders bevorzugten Ausführungsform der Erfindung wird Hydroxyethylstärke an den reduzierenden Endgruppen selektiv oxidiert, indem eine wässrige Lösung aus fraktionierter Hydroxyethylstärke (MG ≤ 10 kDa) zunächst mit einer 0.1 N Iodlösung versetzt wird. Anschließend wird bei Raumtemperatur (RT) 0.1 N KOH Lösung zugegeben, bis die vom Jod stammende Farbe verschwindet. Dieser Schritt wird ein- oder mehrmals wiederholt und die Mischung anschließend für weitere 30 min gerührt. Danach wird die Lösung einer Dialyse unterworfen, wobei die Dialysemembran ein Ausschlußvolumen aufweist, welches das gewünschte Produkt (hier oxidierte Hydroxyethylstärke) im Retenat zurückhält. Nach chromatographischer Reinigung durch eine Kationen-Austauscher Säule wird die Lösung lyophilisiert, wobei die Kationen-Austauschchromatographie auch vor der Dialyse erfolgen kann.

Gemäß einer weiteren besonders bevorzugten Ausführungsform der Erfindung erfolgt die Bindung des Hämoglobins an die selektiv oxidierte Hydroxyethylstärke, indem Hämoglobin zunächst in DMSO oder anderen geeigneten nicht-wässrigen Lösungsmitteln aufgenommen und in eine Dreihals-Rundbodenflasche überführt wird. Dazu wird bei 40°C langsam eine in DMSO aufgenommene Lösung zu einer nach obigen Verfahren oxidierten Hydroxyethylstärke gegeben. Die Reihenfolge dieser Schritte ist jedoch beliebig, Hämoglobin kann also auch zu einer Hydroxyethylstärke-Lösung gegeben werden.

Nach 25-stündigem Rühren bei 40°C wird der Rückstand über Gelpermeationschromatographie (GPC) oder Dialyse und/oder Ultrafiltration gereinigt und so vom Lösungsmittel befreit. Die Molekulargewichtszunahme der Hämoglobinpräparationen läßt sich direkt mittels SDS-PAGE sowie nicht-denaturierender Gelelektrophorese oder aber Ultrazentrifugation (Dichtegradienten- oder Sedimentationsgleichgewichtszentrifugation) ermitteln. Des weiteren eignen sich gängige chromatographische Methoden wie die SEC (Größenausschlußchromatographie) oder Dünnschichtchromatographie (TLC) zur Bestimmung der Molekülgröße. Affinitätschromatographische Verfahren (HIC, RPC) sowie die IEC (Ionenaustauschchromatographie können ebenso die IEF (isolektronische Focussierung) zur Ermittlung modifikationsbedingter Änderungen im physikalischchemischen Molekülverhalten verwendet werden. Der Substitutionsgrad läßt sich durch ¹H-NMR, ¹³C-NMR, Massenspektrometrie bzw. Kapillar-Elektrophorese (CE) quantifizieren. Für diesen Zeck eignet sich ferner die colorimetrische Methode zur Bestimmung freier Aminogruppen in Proteinen mittels TNBS (Habeeb et al., Anal. Biochem., 14, 328 [1966]) in Verbindung mit einem Protein-Test (Bradford, Lowry, Biuret) oder der Stickstoffbestimmung nach Kjeldahl.

Die Erfindung betrifft ebenfalls Sauerstoff-Transport-Mittel, die Hydroxyethylstärke-Hämoglobin-Konjugate und Albumin enthalten. Das Albumin kann dabei menschlichen, tierischen oder rekombinanten Ursprungs sein und wird bevorzugt als wässrige Lösung eingesetzt. Das Sauerstoff-Transport-Mittel enthält Albumin bevorzugt in einer Konzentration von zwischen 2 und 20 g/dL, wobei Konzentrationen zwischen 5 und 15 g/dL bevorzugt sind.

In erfindungsgemäß bevorzugten Sauerstoff-Transport-Mitteln kann das Gewichtsverhältnis von dem Hämoglobin-Hydroxyethylstärke-Konjugat zu Albumin von 1:10 bis 4:1 betragen. Da das Albumin wesentlich preiswerter ist als das Konjugat und zur Erzielung des gewünschten osmotischen Druckes in dem Sauerstoff-Transport-Mittel verwendet werden kann, sind Sauerstoff-Transport-Mittel mit einem vergleichsweise hohen Anteil an Albumin und einem geringen Anteil an Hämoglobin-Hydroxyethylstäke-Konjugaten besonders bevorzugt.

Die Erfindung betrifft ferner Sauerstoff-Transport-Mittel, die Hämoglobin-Hydroxyethylstärke-Konjugate und Albumin enthalten, und eine besonders gute vaskuläre Verträglichkeit aufweisen. Gemäß einer besonders bevorzugten Ausführungsform der Erfindung werden dafür die beschriebenen Konjugate mit Albumin, vorzugsweise mit humanem Serumalbumin vermischt, welches zuvor mit Stickstoffmonoxid gesättigt wurde. Hämoglobin und Albumin besitzen die Eigenschaft NO in N-Nitroso-Form zu komplexieren (vgl. Keaney et al., J. Clin. Invest., 91, (1993) 1582-1589). Vernetzte HBOC-Produkte besitzen in der Regel keine kooperativen Eigenschaften mehr. Daher fehlt ihnen die Fähigkeit zur kooperativen NO-Bindung. Im Rahmen der vorliegenden Erfindung wurde überraschenderweise gefunden, daß dieser Mangel der Hämoglobin-Hydroxyethylstärke-Konjugate kompensiert werden kann, indem ein Sauerstoff-Transport-Mittel verwendet wird, das neben dem Konjugat eine Albuminlösung enthält, die NO komplexiert hat. Dabei erfolgt die Sättigung von Albumin mit NO durch Begasen einer Albuminlösung mit NO unter Sauerstoff-Ausschluß. Die vaskuläre Verträglichkeit des Produkts wird dadurch weiter verbessert.

Die vorliegende Erfindung betrifft insbesondere die Verwendung der erfindungsgemäßen Sauerstoff-Transportmoleküle und der Zusammensetzungen aus den Hämoglobin-Hydroxyethylstärke-Konjugaten und Albumin zur Herstellung eines Arzneimittels zur Verwendung als Blutersatzstoff, Plasma-Expander, Perfusionsmittel, Hämodilutionsmittel und/oder als kardioplegische Lösung.

### BEISPIEL: Herstellung eines Hämoglobin-Hydroxyethylstärke-Konjugates

### A.1 Oxidation der reduzierenden Endgruppen von Hydroxyethylstärke:

Nach einem bevorzugten Verfahren der Erfindung werden die reduzierenden Endgruppen der Hydroxyethylstärke selektiv oxidiert. Zu einer in weniger als 3 ml deionisiertem Wasser aufgenommenen Lösung aus fraktionierter Hydroxyethylstärke (MG ≤ 4 kDa; eingewogene Menge ca. 0,56 mmol) wurden zunächst tropfenweise 2 ml einer 0,1 N Iodlösung gegeben. Anschließend erfolgte die Zugabe von ca. 3,3 ml einer 0,1 N KOH-Lösung bei Raumtemperatur, bis die vom Iod stammende Farbe verschwand. Durch Wiederholung der o.a. Schritte wurden 14 ml Iodlösung und 23 ml KOH-Lösung zu dem Reaktionsansatz gegeben und die Mischung anschließend für weitere 30 min gerührt.

Anschließend wurde die Lösung an einer Kationen-Austauscher-Säule (Amberlite IR 120, H⁺-Form) chromatographisch gereinigt. Nach Diafiltration über eine regenerierte Cellulose-Membran (Millipore PLAC 076 10) mit einer Ausschlußgrenze von 1000 Da wurde die ankonzentrierte Lösung lyophilisiert. Die Kationen-Austausch-Chromatographie kann jedoch auch nach der Diafiltration erfolgen. Die Ausbeute lag in einer Größenordnung von 80-90 %.

### A.2 Alternatives Verfahren zur Oxidation der reduzierenden Endgruppen von Hydroxyethylstärke:

Zu einer in weniger als 3 ml deionisiertem Wasser aufgenommenen Lösung fraktionierter Hydroxyethylstärke (MG ≤ 10 kDa; ca. 5 g) wurden zunächst tropfenweise 2 ml einer 0.1 N Iodlösung gegeben. Anschließend wurde bei Raumtemperatur (RT) 0.1 N KOH Lösung zugegeben, bis die vom Jod stammende Farbe verschwand. Durch Wiederholung des o.a. Schrittes wurden 14 ml Jodlösung und 23 ml KOH-Lösung zu dem Reaktionsansatz gegeben und die Mischung anschließend für weitere 30 min gerührt. Danach wurde die Lösung einer Dialyse mit einem Ausschlußvolumen der Dialysemembran von etwa 9 kDa unterworfen. Nach chromatographischer Reinigung an einer Kationen-Austauscher Säule (Amberlite IR-120) wurde die Lösung lyophilisiert. Die Ausbeute lag in einer Größenordnung von 85%.

### B.1 Desoxygenierung von Hämoglobin durch Begasen:

Bovines Hämoglobin in einer Konzentration von 6 g/dL in 0,5 M NaCl, 0,1 M Na₂HPO₄ und 0,05 M NaHCO₃ wurde durch Begasen desoxygeniert. Das Hämoglobin lag zunächst zu 94 bis 96% in der CO-Form vor. Die Desoxygenierung erfolgte in einem geschlossenen Behälter, in dem die Hämoglobin-Lösung über einen Gasaustauscher zirkuliert wurde, während die Membran kontinuierlich zunächst für eine partielle Oxygenierung mit O₂ und anschließend mit N₂ bei einem Druck von 10 p.s.i. begast wurde. Bei einem Gehalt von 70% Desoxy-Hämoglobin wurde die Desoxygenierung beendet. Anschließend wurde das Hämoglobin unter N₂-Begasung lyophilisiert.

### B.2 Desoxygenierung von Hämoglobin mittels chemischer Reduktionsmittel:

Bovines Hämoglobin in einer Konzentration von 6 g/dL in 0,5 M NaCl, 0,1 M Na₂HPO₄ und 0,05 M NaHCO₃ wurde chemisch reduziert. Dafür wurde die Hämoglobin-Lösung mit 100 mM Na-Disulfit versetzt. Nach einer Stunde bestand die resultierende Lösung zu 75% Desoxy-Hämoglobin. Das Na-Disulfit wurde mittels Ultrafiltration bei einer Membran-Ausschlußgrenze von 50 kDa abgetrennt. Anschließend wurde das Hämoglobin unter N₂-Begasung lyophilisiert.

### C. Kopplung von Hämoglobin an die oxidierten Endgruppen von Hydroxyethylstärke:

Jeweils etwa 1 bis 1,5 g des in den Schritten B.1 und B.2 hergestellten Hämoglobins wurde in 15 ml DMSO aufgenommen und in eine 100 ml Dreihals-Rundbodenflasche überführt. Hierzu wurde bei 40°C langsam eine in 0.5 ml DMSO aufgenommene Lösung von gemäß A. oxidierter Hydroxyethylstärke gegeben. Nach ein- bis zweitägigen Rühren bei 40°C wurde der Rückstand über Dialyse vom Lösungsmittel befreit und mit Hilfe der Diafiltration ankonzentriert. Die Reinheit des Produktes, insbesondere die Abtrennung von Ausgangsprodukten, kann durch Einbeziehung von Standard-Chromatographieverfahren und Ultrafiltration weiterhin verbesert werden.

Der Erfolg der Kopplungsreaktion wurde anhand von Gelpermeationschromatographie nachgewiesen.

## Patentansprüche

1. Sauerstoff-Transport-Mittel enthaltend ein Hämoglobin-Hydroxyethylstärke-Konjugat, **dadurch gekennzeichnet, daß** das Hämoglobin und die Hydroxyethylstärke in dem Konjugat selektiv über Amidbindungen zwischen freien Aminogruppen des Hämoglobins und der in oxidierter Form vorliegenden reduzierenden Endgruppe der Hydroxyethylstärke miteinander verknüpft sind.

2. Sauerstoff-Transport-Mittel nach Anspruch 1, **dadurch gekennzeichnet, daß** das Hämoglobin-Hydroxyethylstärke-Konjugat im Sauerstoff-Transport-Mittel in einer Konzentration zwischen 2 und 40 g/dL vorliegt.

3. Sauerstoff-Transport-Mittel nach Anspruch 2, **dadurch gekennzeichnet, daß** das Hämoglobin-Hydroxyethylstärke-Konjugat im Sauerstoff-Transport-Mittel in einer Konzentration zwischen 5 und 20 g/dL vorliegt.

4. Sauerstoff-Transport-Mittel nach einem der Ansprüche 1 bis 3, **dadurch gekennzeichnet, daß** das Hämoglobin menschlichen, tierischen, pflanzlichen oder rekombinanten Ursprungs ist.

5. Sauerstoff-Transport-Mittel nach einem der Ansprüche 1 bis 4, **dadurch gekennzeichnet, daß** das Hämoglobin bovinen Ursprungs ist.

6. Sauerstoff-Transport-Mittel nach einem der Ansprüche 1 bis 5, **dadurch gekennzeichnet, daß** das Hämoglobin vor der Kopplung an die Hydroxyethylstärke als Desoxy-Hämoglobin oder als Mischung aus Desoxy-Hämoglobin und Hämoglobin in anderen Derivat-Zuständen, wie CO-, O₂ oder Met-Hämoglobin, vorliegt.

7. Sauerstoff-Transport-Mittel nach einem der Ansprüche 1 bis 6, **dadurch gekennzeichnet, daß** das Hämoglobin vor der Kopplung an die Hydroxyethylstärke als Mischung von Desoxy-Hämoglobin und Oxy-Hämoglobin vorliegt, wobei der Anteil von Desoxy-Hämoglobin 20 bis 80% und der Anteil von Hämoglobin in anderen Derivat-Zuständen 80 bis 20% beträgt.

8. Sauerstoff-Transport-Mittel nach einem der Ansprüche 1 bis 7, **dadurch gekennzeichnet, daß** das Hämoglobin vernetztes und/oder polymerisiertes Hämoglobin ist.

9. Sauerstoff-Transport-Mittel nach einem der Ansprüche 1 bis 8, **dadurch gekennzeichnet, daß** die Hydroxyethylstärke ein mittleres Molekulargewicht von 1 bis 40 kDa aufweist.

10. Sauerstoff-Transport-Mittel nach Anspruch 10, **dadurch gekennzeichnet, daß** die Hydroxyethylstärke ein mittleres Molekulargewicht von 2 bis 20 kDa aufweist.

11. Sauerstoff-Transport-Mittel nach einem der Ansprüche 1 bis 10, **dadurch gekennzeichnet, daß** die Hydroxyethylstärke einen molaren Substitutionsgrad von 0,1 bis 0,8 und ein Verhältnis von C₂ : C₆-Substitution im Bereich von 2 bis 20, jeweils bezogen auf die Hydroxyethylgruppen aufweist.

12. Sauerstoff-Transport-Mittel nach einem der Ansprüche 1 bis 11, **dadurch gekennzeichnet, daß** das Mittel ferner Albumin enthält.

13. Sauerstoff-Transport-Mittel nach Anspruch 12, **dadurch gekennzeichnet, daß** das Albumin Serumalbumin menschlichen, tierischen, pflanzlichen oder rekombinanten Ursprungs ist.

14. Sauerstoff-Transport-Mittel nach einem der Ansprüche 12 oder 13, **dadurch gekennzeichnet, daß** das Albumin in einer Konzentration zwischen 2 und 20 g/dL vorliegt.

15. Sauerstoff-Transport-Mittel nach einem der Ansprüche 12 bis 14, **dadurch gekennzeichnet, daß** das Gewichtsverhältnis von dem Hämoglobin-Hydroxyethylstärke-Konjugat zu Albumin im Bereich von 1:10 bis 4:1 liegt.

16. Sauerstoff-Transport-Mittel nach einem der Ansprüche 12 bis 15, **dadurch gekennzeichnet, daß** das Albumin vor Zugabe zu dem Konjugat in wässriger Lösung mit Stickstoffmonoxid (NO) gesättigt vorliegt.

17. Sauerstoff-Transport-Mittel nach einem der Ansprüche 1 bis 16, **dadurch gekennzeichnet, daß** das Mittel als wässrige Lösung oder als Lyophilisat vorliegt.

18. Verfahren zur Herstellung eines Sauerstoff-Transport-Mittels enthaltend ein Hämoglobin-Hydroxyethylstärke-Konjugat, **dadurch gekennzeichnet, daß** man die reduzierenden Endgruppen von Hydroxyethylstärke zunächst oxidiert und anschließend Hämoglobin über freie Aminogruppen mittels Amidbindungen an die oxidierten Endgruppen der Hydroxyethylstärke koppelt.

19. Verfahren nach Anspruch 18, **dadurch gekennzeichnet, daß** man die Oxidation der reduzierenden Endgruppen der Hydroxyethylstärke durchführt, indem man Hydroxyethylstärke zunächst mit einer Iod enthaltenden Lösung vermischt und danach Kaliumhydroxyd-Lösung zugibt.

20. Verfahren nach einem der Ansprüche 18 oder 19, **dadurch gekennzeichnet, daß** man die Verknüpfung der freien Aminogruppen des Hämoglobins mit den in oxidierter Form vorliegenden reduzierenden Endgruppen der Hydroxyethylstärke durchführt, indem man die Einzelkomponenten bei 40 °C vermischt.

21. Verfahren nach einem der Ansprüche 18 bis 20, **dadurch gekennzeichnet, daß** das Hämoglobin menschlichen, tierischen, pflanzlichen oder rekombinanten Ursprungs ist.

22. Verfahren nach einem der Ansprüche 18 bis 21, **dadurch gekennzeichnet, daß** das Hämoglobin bovinen Ursprungs ist.

23. Verfahren nach einem der Ansprüche 18 bis 22, **dadurch gekennzeichnet, daß** das Hämoglobin vor der Kopplung an die Hydroxyethylstärke als Desoxy-Hämoglobin oder als Mischung aus Desoxy-Hämoglobin und Hämoglobin in anderen Derivat-Zuständen, wie CO-, O₂ oder Met-Hämoglobin, vorliegt.

24. Verfahren nach einem der Ansprüche 18 bis 23, **dadurch gekennzeichnet, daß** das Hämoglobin vor der Kopplung an die Hydroxyethylstärke als Mischung von Desoxy-Hämoglobin und Hämoglobin in anderen Derivat-Zuständen vorliegt, wobei der Anteil von Desoxy-Hämoglobin 20 bis 80% und der Anteil von Hämoglobin in anderen Derivat-Zuständen 80 bis 20% beträgt.

25. Verfahren nach einem der Ansprüche 18 bis 24, **dadurch gekennzeichnet, daß** das Hämoglobin vernetztes und/oder polymerisiertes Hämoglobin ist.

26. Verfahren nach einem der Ansprüche 18 bis 25, **dadurch gekennzeichnet, daß** die Hydroxyethylstärke ein mittleres Molekulargewicht von 5 bis 40 kDa, einen molaren Substitutionsgrad von 0,1 bis 0,8 und ein Verhältnis von C₂ : C₆-Substitution im Bereich von 2 bis 20, jeweils bezogen auf die Hydroxyethylgruppen, aufweist.

27. Verwendung eines Sauerstoff-Transport-Mittels nach den Ansprüchen 1 bis 17 oder hergestellt nach den Ansprüchen 18 bis 25 zur Herstellung eines Blutersatzstoffes, Plasma-Expanders, Perfusionsmittels, Hämodilutionsmittels und/oder einer kardioplegischen Lösung.

## Claims

1. Oxygen transfer agent comprising a haemoglobin-hydroxyethylstarch conjugate, **characterized in that** the haemoglobin and the hydroxyethylstarch in the conjugate are linked to one another selectively via amide bonds between free amino groups of the haemoglobin and the reducing end group of the hydroxyethylstarch, which is present in oxidized form.

2. Oxygen transfer agent according to claim 1, **characterized in that** the haemoglobin-hydroxyethylstarch conjugate is present in the oxygen transfer agent in a concentration of between 2 and 20 g/dl.

3. Oxygen transfer agent according to claim 2, **characterized in that** the haemoglobin-hydroxyethylstarch conjugate is present in the oxygen transfer agent in a concentration of between 5 and 20 g/dl.

4. Oxygen transfer agent according to one of claims 1 to 3, **characterized in that** the haemoglobin is of human, animal, vegetable or recombinant origin.

5. Oxygen transfer agent according to one of claims 1 to 4, **characterized in that** the haemoglobin is of bovine origin.

6. Oxygen transfer agent according to one of claims 1 to 5, **characterized in that** before the coupling to the hydroxyethylstarch, the haemoglobin is present as deoxy-haemoglobin or as a mixture of deoxy-haemoglobin and haemoglobin in other derivative states, such as CO- O₂- or met-haemoglobin.

7. Oxygen transfer agent according to one of claims 1 to 6, **characterized in that** before the coupling to the hydroxyethylstarch, the haemoglobin is present as a mixture of deoxy-haemoglobin and oxy-haemoglobin, the content of deoxy-haemoglobin being 20 to 80% and the content of haemoglobin in other derivative states being 80 to 20%.

8. Oxygen transfer agent according to one of claims 1 to 7, **characterized in that** the haemoglobin is crosslinked and/or polymerized haemoglobin.

9. Oxygen transfer agent according to one of claims 1 to 8, **characterized in that** the hydroxyethylstarch has an average molecular weight of 1 to 40 kDa..

10. Oxygen transfer agent according to claim 10 [sic], **characterized in that** the hydroxyethylstarch has an average molecular weight of 2 to 20 kDa..

11. Oxygen transfer agent according to one of claims 1 to 10, **characterized in that** the hydroxyethylstarch has a molar degree of substitution of 0.1 to 0.8 and a ratio of C₂: C₆ substitution in the range from 2 to 20, in each case based on the hydroxyethyl groups.

12. Oxygen transfer agent according to one of claims 1 to 11, **characterized in that** the agent furthermore comprises albumin.

13. Oxygen transfer agent according to claim 12, **characterized in that** the albumin is serum albumin of human, animal, vegetable or recombinant origin.

14. Oxygen transfer agent according to one of claims 12 or 13, **characterized in that** the albumin is present in a concentration of between 2 and 20 g/dl.

15. Oxygen transfer agent according to one of claims 12 to 14, **characterized in that** the weight ratio of the haemoglobin-hydroxyethylstarch conjugate to albumin is in the range from 1:10 to 4:1.

16. Oxygen transfer agent according to one of claims 12 to 15, **characterized in that** the before addition to the conjugate, the albumin is present in an aqueous solution saturated with nitrogen monoxide (NO).

17. Oxygen transfer agent according to one of claims 1 to 16, **characterized in that** the agent is present as an aqueous solution or as a lyophilisate.

18. Process for the preparation of an oxygen transfer agent comprising a haemoglobin-hydroxyethylstarch conjugate, **characterized in that** the reducing end groups of hydroxyethylstarch are first oxidized and haemoglobin is then coupled to the oxidized end groups of the hydroxyethylstarch via free amino groups by means of amide bonds.

19. Process according to claim 18, **characterized in that** the oxidation of the reducing end groups of the hydroxyethylstarch is carried out by first mixing the hydroxyethylstarch with an iodine-containing solution and then adding potassium hydroxide solution.

20. Process according to one of claims 18 or 19, **characterized in that** the linking of the free amino groups of the haemoglobin with the reducing end groups of the hydroxyethylstarch which are present in oxidized form is carried out by mixing the individual components at 40ºC.

21. Process according to one of claims 18 to 20, **characterized in that** the haemoglobin is of human, animal, vegetable or recombinant origin.

22. Process according to one of claims 18 to 21, **characterized in that** the haemoglobin is of bovine origin.

23. Process according to one of claims 18 to 22, **characterized in that** before the coupling to the hydroxyethylstarch, the haemoglobin is present as deoxy-haemoglobin or as a mixture of deoxy-haemoglobin and haemoglobin in other derivative states, such as CO-, O₂- or met-haemoglobin.

24. Process according to one of claims 18 to 23, **characterized in that** before the coupling to the hydroxyethylstarch, the haemoglobin is present as a mixture of deoxy-haemoglobin and haemoglobin in other derivative states, the content of deoxy-haemoglobin being 20 to 80% and the content of haemoglobin in other derivative states being 80 to 20%.

25. Process according to one of claims 18 to 24, **characterized in that** the haemoglobin is crosslinked and/or polymerized haemoglobin.

26. Process according to one of claims 18 to 25, **characterized in that** the hydroxyethylstarch has an average molecular weight of 5 to 40 kDa, a molar degree of substitution of 0.1 to 0.8 and a ratio of C₂: C₆ substitution in the range from 2 to 20, in each case based on the hydroxyethyl groups.

27. Use of an oxygen transfer agent according to claims 1 to 17 or prepared according to claims 18 to 25 for the preparation of a blood substitute, plasma expander, perfusion agent, haemodilution agent and/or cardio-plegic solution.

## Revendications

1. Agent de transport d'oxygène, contenant un conjugué hémoglobine-hydroxyéthylamidon, **caractérisé en ce que** l'hémoglobine et l'hydroxyéthylamidon sont liés l'un à l'autre dans le conjugué sélectivement par des liaisons amide entre des groupes amino libres de l'hémoglobine et le groupe terminal réducteur, se trouvant sous forme oxydée, de l'hydroxyéthylamidon.

2. Agent de transport d'oxygène selon la revendication 1, **caractérisé en ce que** le conjugué hémoglobine-hydroxyéthylamidon est présent dans l'agent de transport d'oxygène à une concentration comprise entre 2 et 40 g/dl.

3. Agent de transport d'oxygène selon la revendication 2, **caractérisé en ce que** le conjugué hémoglobine-hydroxyéthylamidon est présent dans l'agent de transport d'oxygène à une concentration comprise entre 5 et 20 g/dl.

4. Agent de transport d'oxygène selon l'une quelconque des revendications 1 à 3, **caractérisé en ce que** l'hémoglobine est d'origine humaine, animale, végétale ou recombinante.

5. Agent de transport d'oxygène selon l'une quelconque des revendications 1 à 4, **caractérisé en ce que** l'hémoglobine est d'origine bovine.

6. Agent de transport d'oxygène selon l'une quelconque des revendications 1 à 5, **caractérisé en ce que**, avant le couplage à l'hydroxyéthylamidon, l'hémoglobine se trouve sous forme de désoxyhémoglobine ou de mélange de désoxyhémoglobine et d'hémoglobine en d'autres états de dérivés, tels que CO-, O₂- ou Met-hémoglobine.

7. Agent de transport d'oxygène selon l'une quelconque des revendications 1 à 6, **caractérisé en ce que**, avant le couplage à l'hydroxyéthylamidon, l'hémoglobine se trouve sous forme de mélange de désoxyhémoglobine et oxyhémoglobine, la proportion de désoxyhémoglobine allant de 20 à 80 % et la proportion d'hémoglobine en d'autres états de dérivés allant de 80 à 20 %.

8. Agent de transport d'oxygène selon l'une quelconque des revendications 1 à 7, **caractérisé en ce que** l'hémoglobine est une hémoglobine réticulée et/ou polymérisée.

9. Agent de transport d'oxygène selon l'une quelconque des revendications 1 à 8, **caractérisé en ce que** l'hydroxyéthylamidon présente une masse moléculaire moyenne de 1 à 40 kDa.

10. Agent de transport d'oxygène selon la revendication 10, **caractérisé en ce que** l'hydroxyéthylamidon présente une masse moléculaire moyenne de 2 à 20 kDa.

11. Agent de transport d'oxygène selon l'une quelconque des revendications 1 à 10, **caractérisé en ce que** l'hydroxyéthylamidon présente un degré de substitution molaire de 0,1 à 0,8 et un rapport de substitution C₂/C₆ dans la plage de 2 à 20, dans chaque cas par rapportaux groupes hydroxyéthyle.

12. Agent de transport d'oxygène selon l'une quelconque des revendications 1 à 11, **caractérisé en ce que** l'agent contient de l'albumine.

13. Agent de transport d'oxygène selon la revendication 12, **caractérisé en ce que** l'albumine est de l'albumine sérique d'origine humaine, animale, végétale ou recombinante.

14. Agent de transport d'oxygène selon la revendication 12 ou 13, **caractérisé en ce que** l'albumine est présente à une concentration comprise entre 2 et 20 g/dl.

15. Agent de transport d'oxygène selon l'une quelconque des revendications 12 à 14, **caractérisé en ce que** le rapport pondéral du conjugué hémoglobine-hydroxyéthylamidon à l'albumine se situe dans la plage allant de 1:10 à 4:1.

16. Agent de transport d'oxygène selon l'une quelconque des revendications 12 à 15, **caractérisé en ce que**, avant l'addition au conjugué, l'albumine se trouve en solution aqueuse saturée avec du monoxyde d'azote (NO).

17. Agent de transport d'oxygène selon l'une quelconque des revendications 1 à 16, **caractérisé en ce que** l'agent se trouve sous forme de solution aqueuse ou de lyophilisat.

18. Procédé pour la préparation d'un agent de transport d'oxygène contenant un conjugué hémoglobine-hydroxyéthyiamidon, **caractérisé en ce qu'**on oxyde d'abord les groupes terminaux réducteurs de l'hydroxyéthylamidon, et l'hémoglobine est ensuite couplée, par des groupes amino libres, au moyen de liaisons amide, aux groupes terminaux oxydés de l'hydroxyéthylamidon.

19. Procédé selon la revendication 18, **caractérisé en ce qu'**on effectue l'oxydation des groupes terminaux réducteurs de l'hydroxyéthylamidon en mélangeant d'abord de l'hydroxyéthylamidon avec une solution contenant de l'iode, et en y ajoutant ensuite une solution d'hydroxyde de potassium.

20. Procédé selon la revendication 18 ou 19, **caractérisé en ce qu'**on effectue la liaison des groupes amino libres de l'hémoglobine avec les groupes terminaux réducteurs, se trouvant sous forme oxydée, de l'hydroxyéthylamidon, en mélangeant les composants individuels à 40°C.

21. Procédé selon l'une quelconque des revendications 18 à 20, **caractérisé en ce que** l'hémoglobine est d'origine humaine, animale, végétale ou recombinante.

22. Procédé selon l'une quelconque des revendications 18 à 21, **caractérisé en ce que** l'hémoglobine est d'origine bovine.

23. Procédé selon l'une quelconque des revendications 18 à 22, **caractérisé en ce que**, avant le couplage à l'hydroxyéthylamidon, l'hémoglobine se trouve sous forme de désoxyhémoglobine ou de mélange de désoxyhémoglobine et d'hémoglobine en d'autres états de dérivés, tels que CO-, O₂- ou Met-hémoglobine.

24. Procédé selon l'une quelconque des revendications 18 à 23, **caractérisé en ce que**, avant le couplage à l'hydroxyéthylamidon, l'hémoglobine se trouve sous forme de mélange de désoxyhémoglobine et d'hémoglobine en d'autres états de dérivés, la proportion de désoxyhémoglobine allant de 20 à 80 % et la proportion d'hémoglobine en d'autres états de dérivés allant de 80 à 20 %.

25. Procédé selon l'une quelconque des revendications 18 à 24, **caractérisé en ce que** l'hémoglobine est une hémoglobine réticulée et/ou polymérisée.

26. Procédé selon l'une quelconque des revendications 18 à 25, **caractérisé en ce que** l'hydroxyéthylamidon présente une masse moléculaire moyenne de 5 à 40 kDa, un degré de substitution molaire de 0,1 à 0,8 et un rapport de substitution C₂/C₆ dans la plage de 2 à 20, dans chaque cas par rapport aux groupes hydroxyéthyle,

27. Utilisation d'un agent de transport d'oxygène selon les revendications 1 à 17 ou préparé selon les revendications 18 à 25, pour la préparation d'un succédané de sang, d'un succédané de plasma, d'un liquide de perfusion, d'un liquide d'hémodilution et/ou d'une solution cardioplégique.
